# EUROPÄISCHE PATENTANMELDUNG

(11) **EP 4 455 120 A1**
(43) Veröffentlichungstag der Anmeldung: **30.10.2024**
(21) Anmeldenummer: 23170052.7
(22) Anmeldetag: 26.04.2023
(51) Int. Cl.: C07C 67/38, C07C 69/24

(54) **VERFAHREN ZUR ALKOXYCARBONYLIERUNG VON OLEFINEN UNTER EINSATZ VON SYNTHESEGAS**

(71) Anmelder: Evonik Oxeno GmbH & Co. KG, 45772 Marl (DE)
(72) Erfinder: FRANKE, Robert, 45772 Marl (DE); BELLER, Matthias, 18211 Ostseebad Nienhagen (DE); KUCMIERCZYK, Peter, 44628 Herne (DE); JACKSTELL, Ralf, 18106 Rostock (DE); GE, Yao, Chicago (US); NEUMANN, Helfried, 18055 Rostock (DE)
(74) Vertreter: Evonik Patent Association

(57) **Zusammenfassung**

Verfahren zur Alkoxycarbonylierung von Olefinen unter Einsatz von Synthesegas.

## Beschreibung

Die Erfindung betrifft ein Verfahren zur Alkoxycarbonylierung von Olefinen unter Einsatz von Synthesegas (= Gemisch aus CO und H₂).

Die Alkoxycarbonylierung ethylenisch ungesättigter Verbindungen ist ein Prozess mit steigender Bedeutung. Unter einer Alkoxycarbonylierung versteht man die Umsetzung ethylenisch ungesättigter Verbindungen (Olefinen) mit Kohlenmonoxid und Alkoholen in Gegenwart eines Metall-Ligand-Komplexes zu den entsprechenden Estern. Üblicherweise wird als Metall Palladium eingesetzt. Das folgende Schema zeigt die allgemeine Reaktionsgleichung einer Alkoxycarbonylierung:

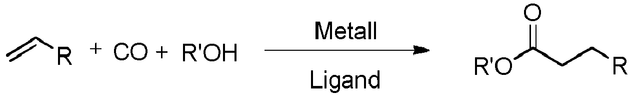

Klassischer Weise wird ein Alkoxycarbonylierungsverfahren unter Einsatz von CO betrieben. Ein solches ist in EP 4 001 256 A1 beschrieben. Die Zufuhr von H₂ wurde bisher absichtlich vermieden da man davon ausgegangen ist, dass diese Nebenreaktion, wie beispielsweise eine Hydroformylierung, fördern würde, und somit der Umsatz zum gewünschten Zielprodukt rückläufig wäre.

Die technische Aufgabe der Erfindung ist die Bereitstellung eines neuen Verfahrens, welches eine gesteigerte Ausbeute liefert.

Die Aufgabe wird gelöst durch ein Verfahren nach Anspruch 1.

Verfahren umfassend die Verfahrensschritte:
a) Vorlegen eines Olefins;
b) Zugabe eines Liganden gemäß Formel (I): 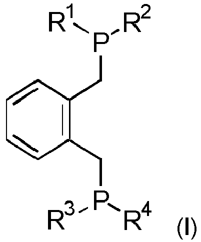 wobei
   R¹ und R³ jeweils für einen -(C₃-C₂₀)-Heteroarylrest stehen,
   R² und R⁴ jeweils für -(C₁-C₁₂)-Alkyl stehen;
c) Zugabe einer Verbindung, welche Pd umfasst;
d) Zugabe eines Alkohols;
e) Zuführen von CO und H₂, wobei der Druck, mit welchem H₂ zugeführt wird mindestens 0,6 MPa (6 bar) beträgt;
f) Erwärmen des Reaktionsgemisches aus a) bis e), wobei das Olefin zu einem Ester umgesetzt wird.

Der Begriff (C₁-C₁₂)-Alkyl umfasst geradkettige und verzweigte Alkylgruppen mit 1 bis 12 Kohlenstoffatomen. Vorzugsweise handelt es sich dabei um (C₁-C₈)-Alkylgruppen, besonders bevorzugt (C₁-C₆)-Alkyl, am meisten bevorzugt (C₁-C₄)-Alkyl.

Der Begriff (C₃-C₂₀)-Heteroaryl umfasst mono- oder polycyclische aromatische Kohlenwasserstoffreste mit 3 bis 20 Kohlenstoffatomen, wobei eins oder mehrere der Kohlenstoffatome durch Heteroatome ersetzt sind. Bevorzugte Heteroatome sind N, O und S. Die (C₃-C₂₀)-Heteroarylgruppen weisen 3 bis 20, bevorzugt 6 bis 14, besonders bevorzugt 6 bis 10 Ringatome auf. Somit ist beispielsweise Pyridyl im Rahmen dieser Erfindung ein C₆-Heteroarylrest, Furyl ist ein C₅-Heteroarylrest.

In einer Variante des Verfahrens wird CO mit einem Druck im Bereich von 1 MPa (10 bar) bis 3 MPa (30 bar) zugeführt.

In einer Variante des Verfahrens wird CO mit einem Druck im Bereich von 1,5 MPa (15 bar) bis 2,5 MPa (25 bar) zugeführt.

In einer Variante des Verfahrens wird CO mit einem Druck von 2 MPa (20 bar) zugeführt.

In einer Variante des Verfahrens wird H₂ mit einem Druck im Bereich von 0,6 MPa (6 bar) bis 2,9 MPa (29 bar) zugeführt.

In einer Variante des Verfahrens wird H₂ mit einem Druck im Bereich von 0,9 MPa (9 bar) bis 2,1 MPa (21 bar) zugeführt.

In einer Variante des Verfahrens wird H₂ mit einem Druck im Bereich von 1 MPa (10 bar) bis 2 MPa (20 bar) zugeführt.

In einer Variante des Verfahrens liegt das Verhältnis der Drücke, mit welchem CO und H₂ zugeführt werden, im Bereich von 1:0,3 bis 1:1,4.

In einer Variante des Verfahrens sind R¹, R³ jeweils ausgewählt aus Furyl, Thienyl, Pyrrolyl, Oxazolyl, Isoxazolyl, Thiazolyl, Isothiazolyl, Imidazolyl, Pyrazolyl, Furazanyl, Tetrazolyl, Pyridyl, Pyridazinyl, Pyrimidyl, Pyrazinyl, Benzofuranyl, Indolyl, Isoindolyl, Benzimidazolyl, Chinolyl, Isochinolyl.

In einer Variante des Verfahrens stehen R² und R⁴ für *^{ter}*Bu.

In einer Variante des Verfahrens weist der Ligand in Verfahrensschritt b) die Formel (1) auf:

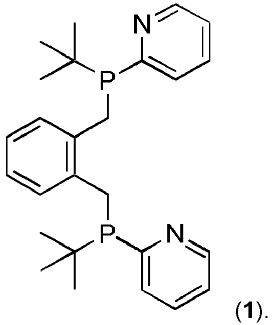

In einer Variante des Verfahrens ist die Verbindung in Verfahrensschritt c), welche Pd umfasst, ausgewählt aus Palladiumdichlorid, Palladium(II)-acetylacetonat, Palladium(II)-acetat, Dichloro(1,5-cyclooctadiene)palladium(II), Bis(dibenzylideneaceton)palladium, Bis(acetonitrile)dichloropalladium(ll), Palladium(cinnamyl)dichlorid.

In einer Variante des Verfahrens ist die Verbindung in Verfahrensschritt c), welche Pd umfasst, Pd(acac)₂.

In einer Variante des Verfahrens ist der Alkohol in Verfahrensschritt d) ausgewählt aus Methanol, Ethanol, 1-Propanol, 1-Butanol, 1-Pentanol, 1-Hexanol, 2-Propanol, *tert*-Butanol, 3-Pentanol, Cyclohexanol, Phenol, oder Mischungen davon.

In einer Variante des Verfahrens ist der Alkohol in Verfahrensschritt d) Methanol.

In einer Variante des Verfahrens umfasst das Verfahren den zusätzlichen Verfahrensschritt d`) umfasst:
d') Zugabe einer Säure, welche ausgewählt ist aus: Aluminiumtriflat, Schwefelsäure, Methylsulfonsäure (MSA), para-Toluolsulfonsäure (p-TSA).

In einer Variante des Verfahrens wird in Verfahrensschritt d') Aluminiumtriflat zugegeben.

In einer Variante des Verfahrens liegt das Verhältnis Säure : Ligand im Bereich von 1 mol : 1 mol bis 15 mol : 1 mol.

In einer Variante des Verfahrens liegt das Verhältnis Aluminiumtriflat : Ligand im Bereich von 1 mol: 1 mol bis 15 mol: 1 mol.

Die Reaktionsmischung wird in Verfahrensschritt f) des erfindungsgemäßen Verfahrens vorzugsweise auf eine Temperatur im Bereich von 30 °C bis 150 °C, bevorzugt von 40 °C bis 140 °C, besonders bevorzugt von 50 °C bis 120 °C erwärmt, um das Olefin zu einem Ester umzusetzen.

Im Folgenden soll die Erfindung anhand eines Ausführungsbeispiels näher erläutert werden.

### Experiment 1

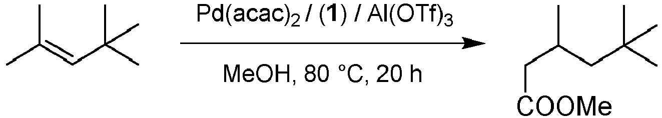

Pd (acac)₂: 0.04 mol%
(1): 0,12 mol%
Al(OTf)₃: 0,6 mol%

Der Versuch wurde einmal mit Synthesegas und als Vergleichsversuch mit reinem CO durchgeführt. Das Synthesegas war hierbei ein Gemisch aus CO und H₂. CO wurde in beiden Versuchen mit einem Druck von 20 bar zugeführt. Bei dem Versuch mit Synthesegas wurde das H₂ ebenfalls mit 20 bar zugeführt, womit sich ein Gesamtdruck von 40 bar eingestellt hat.

### Ausbeute an Ester:

CO: 47 %
CO + H₂: 67 %

### Experiment 2 (Variation des H₂-Drucks)

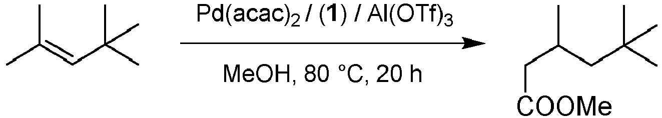

Pd (acac)₂: 0.04 mol%
(1): 0,12 mol%
Al(OTf)₃: 0,6 mol%

Die Versuchsreihe wurde mit einem konstanten CO-Druck von 20 bar durchgeführt. Der H₂-Druck wurde entsprechend der nachfolgenden Tabelle variiert.

| p(H₂) [bar] | Ausbeute [%] |
|---|---|
| 0 | 47 |
| 5 | 44 |
| 10 | 63 |
| 20 | 67 |
| 30 | 41 |
| 40 | 41 |

Wie die durchgeführten Versuche zweigen, konnte die Ausbeute durch den Einsatz von Synthesegas gesteigert werden.

## Patentansprüche

1. Verfahren umfassend die Verfahrensschritte:
a) Vorlegen eines Olefins;
b) Zugabe eines Liganden gemäß Formel (I): 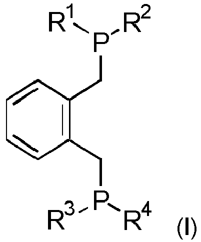 wobei
R¹ und R³ jeweils für einen -(C₃-C₂₀)-Heteroarylrest stehen,
R² und R⁴ jeweils für -(C₁-C₁₂)-Alkyl stehen;
c) Zugabe einer Verbindung, welche Pd umfasst;
d) Zugabe eines Alkohols;
e) Zuführen von CO und H₂, wobei der Druck, mit welchem H₂ zugeführt wird mindestens 0,6 MPa (6 bar) beträgt;
f) Erwärmen des Reaktionsgemisches aus a) bis e), wobei das Olefin zu einem Ester umgesetzt wird.

2. Verfahren nach Anspruch 1,
wobei CO mit einem Druck im Bereich von 1 MPa (10 bar) bis 3 MPa (30 bar) zugeführt wird.

3. Verfahren nach einem der Ansprüche 1 oder 2,
wobei CO mit einem Druck im Bereich von 1,5 MPa (15 bar) bis 2,5 MPa (25 bar) zugeführt wird.

4. Verfahren nach einem der Ansprüche 1 bis 3,
wobei H₂ mit einem Druck im Bereich von 0,6 MPa (6 bar) bis 2,9 MPa (29 bar) zugeführt wird.

5. Verfahren nach einem der Ansprüche 1 bis 4,
wobei H₂ mit einem Druck im Bereich von 0,9 MPa (9 bar) bis 2,1 MPa (21 bar) zugeführt wird.

6. Verfahren nach einem der Ansprüche 1 bis 5,
wobei das Verhältnis der Drücke, mit welchem CO und H₂ zugeführt werden, im Bereich von 1:0,3 bis 1:1,4 liegt.

7. Verfahren nach einem der Ansprüche 1 bis 6,
wobei R¹, R³ jeweils ausgewählt sind aus Furyl, Thienyl, Pyrrolyl, Oxazolyl, Isoxazolyl, Thiazolyl, Isothiazolyl, Imidazolyl, Pyrazolyl, Furazanyl, Tetrazolyl, Pyridyl, Pyridazinyl, Pyrimidyl, Pyrazinyl, Benzofuranyl, Indolyl, Isoindolyl, Benzimidazolyl, Chinolyl, Isochinolyl.

8. Verfahren nach einem der Ansprüche 1 bis 7,
wobei R² und R⁴ für *^{ter}*Bu stehen.

9. Verfahren nach einem der Ansprüche 1 bis 8,
wobei der Ligand in Verfahrensschritt b) die Formel (**1**) aufweist:

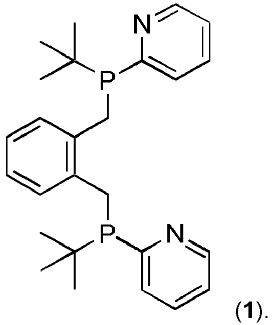

10. Verfahren nach einem der Ansprüche 1 bis 9,
wobei die Verbindung in Verfahrensschritt c), welche Pd umfasst, ausgewählt ist aus Palladiumdichlorid, Palladium(II)-acetylacetonat, Palladium(II)-acetat, Dichloro(1,5-cyclooctadiene)palladium(ll), Bis(dibenzylideneaceton)palladium, Bis(acetonitrile)dichloropalladium(ll), Palladium(cinnamyl)dichlorid

11. Verfahren nach einem der Ansprüche 1 bis 10,
wobei die Verbindung in Verfahrensschritt c), welche Pd umfasst, Pd(acac)₂ ist.

12. Verfahren nach einem der Ansprüche 1 bis 11,
wobei der Alkohol in Verfahrensschritt d) ausgewählt ist aus Methanol, Ethanol, 1-Propanol, 1-Butanol, 1-Pentanol, 1-Hexanol, 2-Propanol, *tert*-Butanol, 3-Pentanol, Cyclohexanol, Phenol, oder Mischungen davon.

13. Verfahren nach einem der Ansprüche 1 bis 12,
wobei der Alkohol in Verfahrensschritt d) Methanol ist.

14. Verfahren nach einem der Ansprüche 1 bis 13,
wobei das Verfahren den zusätzlichen Verfahrensschritt d`) umfasst:
d`) Zugabe einer Säure, welche ausgewählt ist aus: Aluminiumtriflat, Schwefelsäure, Methylsulfonsäure (MSA), para-Toluolsulfonsäure (p-TSA).

15. Verfahren nach Anspruch 14,
wobei das Verhältnis Säure : Ligand im Bereich von 1 mol : 1 mol bis 15 mol : 1 mol liegt.
